# EUROPEAN PATENT SPECIFICATION

(11) **EP 1 638 971 B1**
(45) Date of publication and mention of the grant of the patent: **20.08.2008**
(21) Application number: 04734570.7
(22) Date of filing: 24.05.2004
(51) Int. Cl.: C07D 471/08

(54) **PREPARATION OF SUBSTITUTED QUINOXALINES FROM THE DIANILINE WITH 2,3-DIHYDROXY-1,4-DIOXANE**
HERSTELLUNG VON SUBSTITUIERTEN CHINOXALINEN AUS DEN DIANILINEN MIT 2,3-DIHYDROXY-1,4-DIOXAN
PREPARATION DE QUINOXALINES SUBSTITUEES A PARTIR DE LA DIANILINE AVEC DU 2,3-DIHYDROXY-1,4-DIOXANE

(30) Priority: 04.06.2003 US 475589 P
(43) Date of publication of application: 29.03.2006
(73) Proprietor: Pfizer Products Inc., Groton, CT 06340 (US)
(72) Inventor: RAINVILLE, Joseph, Philip, Groton, CT 06340 (US); TABER, Geraldine, Patricia, Groton, CT 06340 (US)
(74) Representative: Duncan, Garreth Andrew
(86) International application number: PCT/IB2004/001787
(87) International publication number: WO 2004/108725

(56) References cited:
- US-B1- 6 410 550
- VENUTI, MICHAEL C.: "2,3-Dihydroxy-1,4-dioxane: A Stable Synthetic Equivalent of Anhydrous Glyoxal" SYNTHESIS, vol. 1, 1982, pages 61-63, XP002291747
- REWCASTLE, G. W., ET AL.: "Tyrosine Kinase Inhibitors." J. MED. CHEM., vol. 39, no. 4, 1996, pages 918-928, XP002291748

## Description

### Background of the Invention

The present invention comprises a new process for the preparation of substituted quinoxalines by cyclization of the corresponding dianiline.

More particularly, the present invention relates to a new process for the preparation of aryl fused azapolycyclic compounds having the formula 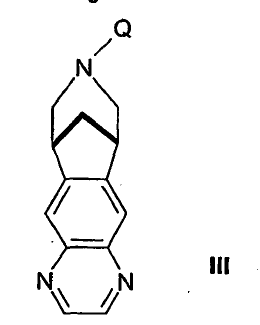 wherein Q is a nitrogen protecting group.

The synthesis, composition, and methods of use of certain aryl fused azapolycyclic compounds of formula III are disclosed in United States Patent No. 6,410,550 which is incorporated herein by reference therein in its entirety.

The foregoing patent discloses the formation of compounds of formula III through the cyclization of the corresponding dianiline with aqueous glyoxal. Improvements in purity were needed in order to provide a more cost effective process.

### Summary of the Invention

The present invention comprises a process for preparing a compound having the formula 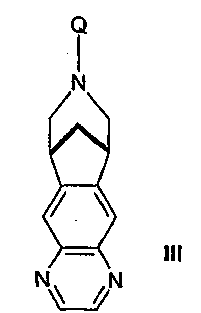 from a compound of the formula 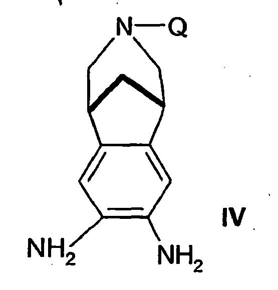 wherein Q is a nitrogen protecting group.

The cyclization of compound IV into compound III is illustrated below 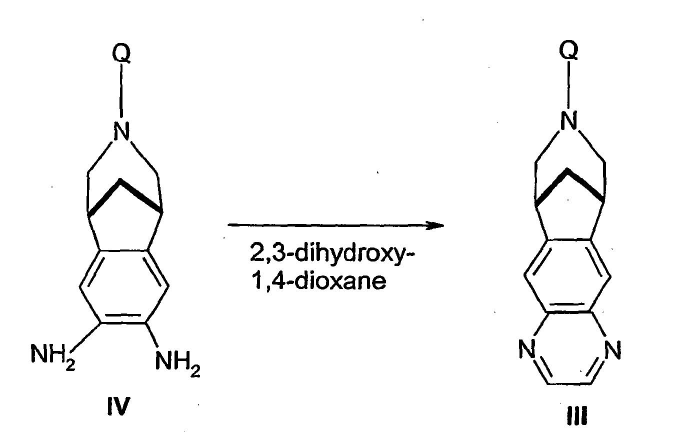 The cyclization is preferably carried out at a temperature range of from about 20°C to about 25°C for a period of about 1 to about 25 hours and more preferably for about 1 to about hours.

Suitable inert solvents are selected from the group consisting of aqueous alcohol, dioxane, tetrahydrofuran, DMF, DMSO, toluene and ethyl acetate. Preferably the solvent is aqueous isopropanol.

The nitrogen protecting group Q is preferably a trifluoroacetyl group or a *t*-butoxy carbonyl group. More preferably Q is a trifluoroacetyl group.

Compounds of formula IV can be prepared by the synthetic method which is described and referred to in United States Patent No. 6,410,550.

As used herein, the expression "inert solvent" refers to a solvent system in which the components do not interact with starting materials, reagents, or intermediates of products in a manner that adversely affects the yield of the desired product.

### Detailed Description of the Invention

The present invention provides a new process for the preparation of substituted quinoxalines (III) by cyclizing the corresponding dianiline (IV) with 2,3-dihydroxy-1,4-dioxane. The synthesis of compounds of formula (IV) is disclosed in United States Patent No 6,410,550.

In particular, the present invention provides an alternative route to benzazepines of formula III in high purity and yield. Prior attempts, as disclosed in United States Patent No. 6,410,550, to convert compounds of formula IV into compounds of formula III utilized either 40% aqueous glyoxal or the addition adduct of sodium bisulfite and ethane dione. Both of these reactions required purification steps.

The cyclization agent employed in the present invention should provide ease of handling, better stoichiometric accuracy, control of self-polymerization and reactivity at ambient temperatures.

Compounds of formula III are precursors to the aryl fused azapolycyclic compound of formula V and its pharmaceutical acceptable acid salts as illustrated below. Preferably the acid salt is the L-tartaric acid salt. The compound of formula V is useful in the treatment of central nervous system disorders as described below.

Removal of the nitrogen protecting group Q is carried out with methods well known in the art, such as, by heating in aqueous methanol in the presence of sodium carbonate. 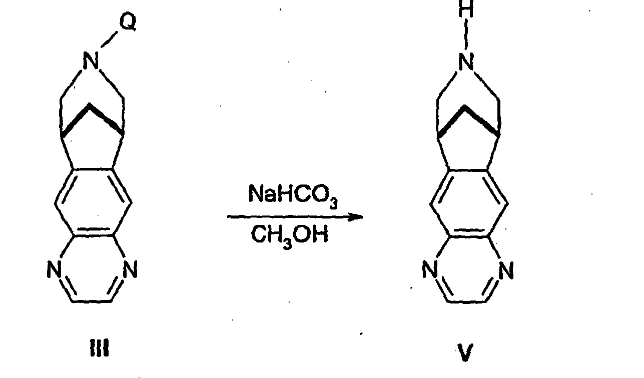 wherein Q is as defined above.

Compounds of formula V bind to neuronal nicotinic acetylcholine specific receptor sites and are useful in modulating cholinergic function. Such compounds are useful in the treatment of inflammatory bowel disease (including but not limited to ulcerative colitis, pyoderma gangrenosum and Crohn's disease), imitable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, hypertension, bulimia, anorexia, obesity, cardiac arrythmias, gastric acid hypersecretion, ulcers, pheochromocytoma, progressive supranuclear palsy, chemical dependencies and addictions (e.g., dependencies on, or addictions to nicotine (and/or tobacco products), alcohol, benzodiazepines, barbiturates, opioids or cocaine), headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, including petit mal absence epilepsy, senile dementia of the Alzheimer's type (AD), Parkinson's disease (PD), attention deficit hyperactivity disorder (ADHD) and Tourette's Syndrome.

The compounds of the formula V and their pharmaceutically acceptable salts (hereafter "the active compounds") can be administered via either the oral, transdermal (e.g., through the use of a patch), intranasal, sublingual, rectal, parenteral or topical routes. Transdermal and oral administration are preferred. These compounds are, most desirably, administered in dosages ranging from about 0.01 mg up to about 1500 mg per day, preferably from about 0.1 to about 300 mg per day in single or divided doses, although variations will necessarily occur depending upon the weight and condition of the subject being treated and the particular route of administration chosen. However, a dosage level that is in the range of about 0.001 mg to about 10 mg per kg of body weight per day is most desirably employed. Variations may nevertheless occur depending upon the weight and condition of the persons being treated and their individual responses to said medicament, as well as on the type of pharmaceutical formulation chosen and the time period and interval during which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds can be administered alone or in combination with pharmaceutically acceptable carriers or diluents by any of the several routes previously indicated. More particularly, the active compounds can be administered in a wide variety of different dosage forms, e.g., they may be combined with various pharmaceutically acceptable inert carriers in the form of tablets, capsules, transdermal patches, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. In addition, oral pharmaceutical compositions can be suitably sweetened and/or flavored. In general, the active compounds are present in such dosage forms at concentration levels ranging from about 5.0% to about 70% by weight.

For oral administration, tablets may contain a variety of excipients, disintegrants, lubricating agents, and fillers. Aqueous suspensions for oral administration may be combined with flavor, coloring matter, and diluents.

For parental administration, a solution of the active compound may be suitably buffered and may be diluted with a vegetable oil or propylene glycol.

The following examples are provided for the purpose of further illustration and are not intended to limit the scope of the claimed invention.

### EXAMPLES 1

### Cyclization of 2,3,4,5-tetrahydro-3-(trifluoroacetyl-1,5-methano-1H-3-benzazepine-7,8-diamine (compound IV) with 2,3-dihyroxy-1,4-dioxane to form 7,8,9,10-tetrahvdro-8-(trifluoroacetyl)-6,10-methano-6H-pyrazino[2,3-h][3]benzazepine (compound III).

To a 200ml aqueous isopropanolic solution (80:20 IPO:H2O) of compound II (8.2g, 28.9mmol), an equimolar quantity of 2,3-dihydroxy-1,4-dioxane is added and the mixture stirred at 20-25°C for 15 hours. The solution is concentrated by distillation to 5mL/g of compound II, and cooled. Water is added and the product is granulated at ambient temperature then isolated by vacuum filtration.

Yield: 7.9g in 89%.

Purity: High Performance Liquid Chromatography (HPLC) 99.2 weight % versus external standard

Melting Point: 171.5°C

### EXAMPLE II

### Deprotection of 7,8,9,10-tetrahydro-8-(trifluoroacetyl)-6,10-methano-6H-pyrazino[2,3-h][3]benzazepine (compound IV) to form 7,8,9,10-tetrahydro-6,10-methano-6H-pyrazino[2,3-h][3]benzazepine (compound V)

13.4g, 335mmol of sodium hydroxide pellets are dissolved in 335mL of process water. To this, 33.21g, 108mmol of compound I and 83ml of methylene chloride (2.5mL/g of compound I) are added and redacted at 20-25°C for 3 to 4. hours. Upon reaction completion, 250mL of methylene chloride (7.5mL/g of compound I) are added and the mixture is stirred, settled and separated. The product rich methylene chloride is displaced with methanol and treated with 25 w/w % Darco Kbb carbon. The carbon is filtered away and the methanolic free base solution of compound III is treated with a methanolic solution of L-tartaric acid 1.1 molar equivalents. The product slurry is granulated at ambient temperature and filtered to give the L-tartaric acid salt of compound III (7,8,9,10-tetrahydro-6,10-methano-6H-pyrazino[2,3-h][3]benzazepine, (2R,3R)-2,3-dihydroxybutanedioate (1:1))

Yield: 31.2g in 80%

Purity: HPLC >99.0 weight % versus external standard

## Claims

1. A process for preparing a chemical moiety wherein said chemical moiety is a compound having the formula 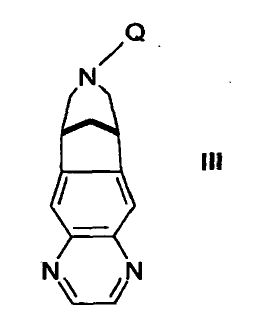 comprising the step of cyclizing a chemical moiety wherein said chemical moiety is a compound having the formula 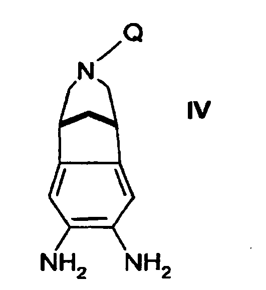 wherein Q is a nitrogen protecting group,
with 2,3-dihydroxy-1,4- dioxane in an inert solvent.

2. A process according to claim 1 wherein said cyclization is conducted at a temperature range of from about 20°C to about 25°C for a period of from about 1 to about 26 hours.

3. A process according to claim 1 wherein said inert solvent is selected from the group consisting of aqueous alcohol, dioxane, tetrahydrofuran, DMF, DMSO, toluene, and ethyl acetate.

4. A process according to claim 3 wherein said inert solvent is aqueous isopropanol.

5. A process according to claim 1 wherein the nitrogen protecting group Q is a trifluoroacetyl group or a-t-butoxycarbonyl group.

6. A process according to claim 5 wherein the nitrogen protecting group is a trifluoroacetyl group.

7. A process according to claim 2 wherein said cyclization is conducted for a period of from about 1 to about 4 hours.

## Patentansprüche

1. Verfahren zum Herstellen einer chemischen Gruppierung, wobei die chemische Gruppierung eine Verbindung mit der Formel 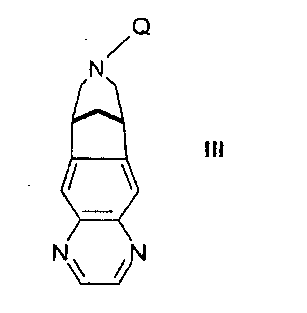 ist, umfassend die Stufe des Zyklisierens einer chemischen Gruppierung, wobei die chemische Gruppierung eine Verbindung mit der Formel 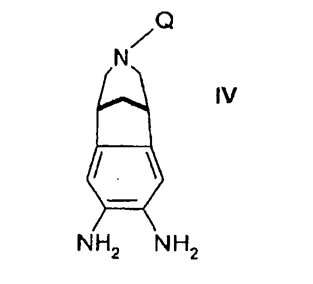 ist, worin Q eine Stickstoffschutzgruppe ist, mit 2,3-Dihydroxy-1,4-dioxan in einem inerten Lösungsmittel.

2. Verfahren gemäß Anspruch 1, wobei die Zyklisierung in einem Temperaturbereich von etwa 20°C bis etwa 25°C für eine Dauer von etwa 1 bis etwa 25 Stunden durchgeführt wird.

3. Verfahren gemäß Anspruch 1, wobei das inerte Lösungsmittel ausgewählt ist aus der Gruppe, bestehend aus wässrigem Alkohol, Dioxan, Tetrahydrofuran, DMF, DMSO, Toluol und Ethylacetat.

4. Verfahren gemäß Anspruch 3, wobei das inerte Lösungsmittel wässriges Isopropanol ist.

5. Verfahren gemäß Anspruch 1, wobei die Stickstoffschutzgruppe Q eine Trifluoracetylgruppe oder eine t-Butoxycarbonylgruppe ist.

6. Verfahren gemäß Anspruch 5, wobei die Stickstoffschutzgruppe eine Trifluoracetylgruppe ist.

7. Verfahren gemäß Anspruch 2, wobei die Zyklisierung für eine Dauer von etwa 1 bis etwa 4 Stunden durchgeführt wird.

## Revendications

1. Procédé pour la préparation d'un groupement chimique, dans lequel ledit groupement chimique est un composé répondant à la formule 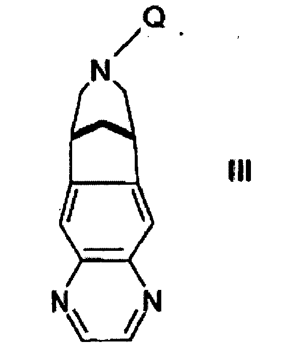 comprenant l'étape de cyclisation d'un groupement chimique, ledit groupement chimique étant un composé répondant à la formule 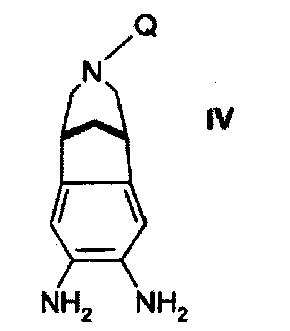 dans laquelle Q représente un groupe protecteur de l'atome d'azote,
avec du 2,3-dihydroxy-1,4-dioxanne dans un solvant inerte.

2. Procédé suivant la revendication 1, dans lequel ladite cyclisation est effectuée à une température d'environ 20°C à environ 25°C pendant une période d'environ 1 à environ 25 heures.

3. Procédé suivant la revendication 1, dans lequel ledit solvant inerte est choisi dans le groupe consistant en une solution aqueuse d'un alcool, le dioxanne, le tétrahydrofuranne, le DMF, le DMSO, le toluène et l'acétate d'éthyle.

4. Procédé suivant la revendication 3, dans lequel ledit solvant inerte est une solution aqueuse d'isopropanol.

5. Procédé suivant la revendication 1, dans lequel le groupe Q protecteur de l'atome d'azote est un groupe trifluoracétyle ou un groupe tertiobutoxycarbonyle.

6. Procédé suivant la revendication 5, dans lequel le groupe protecteur de l'atome d'azote est un groupe trifluoracétyle.

7. Procédé suivant la revendication 2, dans lequel ladite cyclisation est effectuée pendant une période d'environ 1 à environ 4 heures.
